# EUROPEAN PATENT APPLICATION

(11) **EP 1 464 282 A2**
(43) Date of publication of application: **06.10.2004**
(21) Application number: 04003114.8
(22) Date of filing: 12.02.2004
(51) Int. Cl.: A61B 5/15, A61B 5/145

(54) **Endcap for lancing device and method of use**

(30) Priority: 19.02.2003 US 447818 P
(71) Applicant: Bayer HealthCare, LLC, Tarrytown, New York 10591 (US)
(72) Inventor: Kheiri, Mohammad A., Elkhart Indiana 46514 (US)
(74) Representative: Burkert, Frank, Dr.

(57) **Abstract**

An endcap (12) for a lancing device (10) includes a contact face (26) with a pattern of ribs (28) or pressure points used to massage skin around a lancet site to force blood for testing from a puncture at the lanced site. The pattern of ribs includes channels (30) defined between the ribs which do not apply pressure on the skin during massaging such that blood flows under the skin below the channels to the puncture. Massaging is performed by applying the contact face under constant pressure to the skin, rocking the contact face, rotating the contact face, or pumping the contact face.

## Description

### FIELD OF THE INVENTION

The present invention relates generally to blood monitoring devices, and, more particularly to endcaps for blood monitoring devices which have a face with a rib pattern for massaging skin before or after a puncture is made by a lance on alternate sites of the body to cause flow of blood from the puncture.

### BACKGROUND OF THE INVENTION

It is often necessary to quickly and painlessly obtain a sample of blood and perform an analysis of the blood sample. One example of a need for painlessly obtaining a sample of blood is in connection with a blood glucose monitoring system where a user must frequently use the system to monitor the user's blood glucose level.

Those who have irregular blood glucose concentration levels are medically required regularly to self-monitor their blood glucose concentration level. An irregular blood glucose level can be brought on by a variety of reasons including illness such as diabetes. The purpose of monitoring the blood glucose concentration level is to determine the blood glucose concentration level and then to take corrective action, based upon whether the level is too high or too low, to bring the level back within a normal range. The failure to take corrective action can have serious implications. When blood glucose levels drop too low - a condition known as hypoglycemia - a person can become nervous, shaky, and confused. That person's judgment may become impaired and they may eventually faint. A person can also become very ill if their blood glucose level becomes too high - a condition known as hyperglycemia. Both conditions, hypoglycemia and hyperglycemia, are potentially life-threatening emergencies.

One method of monitoring a person's blood glucose level is with a portable, hand-held blood glucose testing device. In order to check the blood glucose level with the testing device, a drop of blood is obtained from the fingertip using a lancing device. A typical lancing device contains a needle lancet to puncture the skin of a finger. This monitoring procedure often is repeated several times a day. Because this procedure can be painful, instruments have been developed to obtain blood from alternate sites other than a fingertip such as arms, legs and palm of the hand. Although obtaining a blood sample from an alternate site is less painful, it usually is very slow. Consequently, available instruments use vacuum or pressure to accelerate drawing blood from a puncture at an alternate site. These devices often cause a hematoma at the site. Other devices use a compression ring which chokes blood supply at the puncture. These procedures are known to cut off blood flow through very fine capillaries typically found under the skin at these alternate sites.

### SUMMARY OF THE INVENTION

The present invention is an endcap for a lancing device used to lance skin in order to draw blood for testing. The endcap allows the lancing device to lance a primary site such as a fingertip or an alternate site such as an arm painlessly and quickly. The endcap has a face with a pattern of pressure points or ribs. The pattern includes channels or gaps between the pressure points or ribs.

With the endcap on the lancing device, the face of the endcap is pressed onto the skin at the chosen site. By massaging the skin with the pattern of ribs or pressure points by rocking, turning, plunging or pressing the endcap on the skin or combining more than one technique, blood is caused to flow under the channels to the puncture. The endcap can be molded of transparent material allowing the user to observe the puncture and the amount of blood drawn to the skin surface.

### BRIEF DESCRIPTION OF THE FIGURES

Other objects and advantages of the invention will become apparent upon reading the following detailed description in conjunction with the drawings in which:
FIG. 1 is an enlarged perspective view of a lancing device with an endcap constructed in accordance with a first embodiment of the present invention;
FIG. 2 is an enlarged perspective view of a second embodiment of the present invention;
FIG. 3 is an enlarged perspective view of a third embodiment of the present invention;
FIG. 4 is an enlarged perspective view of a fourth embodiment of the present invention;
FIG. 5 is an enlarged perspective view of a fifth embodiment of the present invention;
FIG. 6 is an enlarged perspective view of a sixth embodiment of the present invention;
FIG. 7 is an enlarged perspective view of an seventh embodiment of the present invention;
FIG. 8 is an enlarged perspective view of a eighth embodiment of the present invention;
FIG. 9 is an enlarged perspective view of a ninth embodiment of the present invention;
FIG. 10 is an enlarged perspective view of an tenth embodiment of the present invention;
FIG. 11 is an enlarged perspective view of a eleventh embodiment of the present invention; and
FIG. 12 is a cross sectional view of the endcap taken along line 12-12 in FIG. 1.

### DETAILED DESCRIPTION OF THE PREFERRED EMBODIMENTS

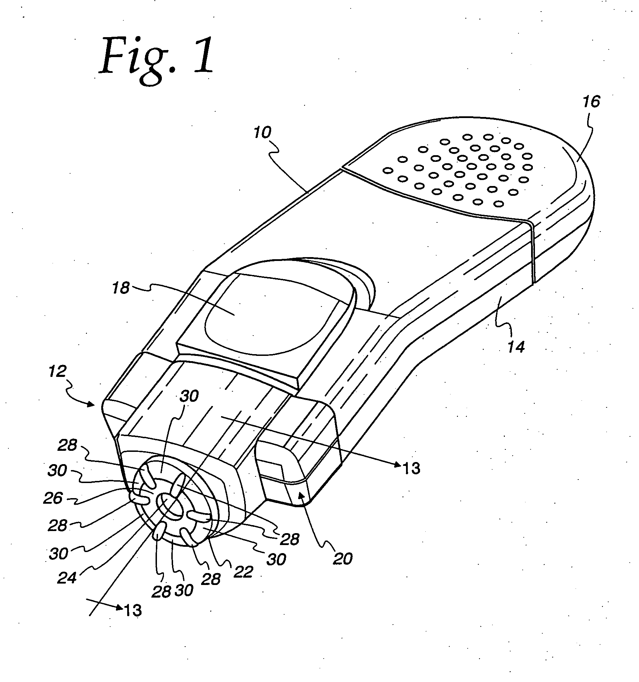

Many children and adults must draw their blood several times a day to perform an analysis of the blood sample drawn. The blood is drawn using a lancing device. An example of a known lancing device 10 with an endcap 12 constructed in accordance with the principles of the present invention is shown in FIG. 1. The known lancing device 10 is described in United States Patent No. 5,954,738 and this patent is incorporated herein in its entirety by reference. The lancing device 10 includes a main housing 14 and a movable housing 16 movable relative to the main housing 14. Internally, the lancing device 10 includes a lance to puncture skin and a driving mechanism for driving the lance out of the endcap 12 and into a patient's skin. The driving mechanism is cocked by pulling the movable housing 16 away from the main housing 14. The driving mechanism is fired by pushing a button 18. In accordance with the present invention, the lancing device 10 is provided with the alternate site endcap 12. Through the use of the endcap 12, the lancing device 10 can be used to obtain blood samples from sites on a patient's body other than a fingertip, although the endcap 12 also allows blood to be drawn from a fingertip if desired. Obtaining blood samples from alternative sites such as an arm or leg reduces the pain experienced by the patient.

The endcap 12 includes a basecap 20 that is releasably secured to the lancing device 10. The eridcap 12 also includes a top cap 22 which has a center aperture 24 for the passage of the lance in the lancing device 10. The top cap has a concave face (chamfer) 26 that is sloped toward the aperture 24 (FIG. 13).

The face 26 of the top cap 20 includes a pattern of six ribs or pressure points 28. The ribs 28 are of a height to accomplish or massage as described hereinafter. A height of .030 inch to .060 inch has been found effective. The pattern includes gaps or channels 30 between adjacent ribs 28. The ribs 28 are also chamfered parallel to the concave face.

In use, the lancing device 10 is placed on an alternate site such as an arm or leg with the face 26 of the top cap 22 against the skin at the site. The pattern of ribs 28 provide pressure points on the skin around the puncture site while the gaps or channels 30 between the ribs 28 define areas of no pressure on the skin. These areas of no pressure act as channels under the skin for blood flow toward a puncture in the skin formed by the lance in the lancing device 10.

After the lance has been driven into the skin and a puncture made, the lancing device 10 is held on the skin of the patient with the face 26 of the top cap 22 against the skin. The skin is then massaged by one of four techniques or by combining more than one technique to cause blood to flow to the puncture where blood can be drawn and tested. These massage techniques can also be applied before lancing the skin to prepare for better blood flow. One massage technique is to apply gentle pressure to the skin while alternately rotating the end cap 12 clockwise and counterclockwise. A second massage technique is to rock the top cap 22. A third massage technique is repeatedly to apply and release pressure on the puncture site by up and down motion of the top cap 22. A fourth massage technique is to apply continuous pressure on the puncture site for five to twenty seconds after lancing by holding down on the top cap 22. In all these techniques, the endcap 12 has to be kept firmly on one place. Each of these massage techniques causes blood to flow to the puncture, and can be performed before lancing, after lancing or before and after lancing. These massage techniques can be performed individually or in combination. The ribs 28 also serve to grip the skin of the patient during massaging making it easier to keep the lancing device 10 over the puncture site which reduces the chance of smearing the blood sample.

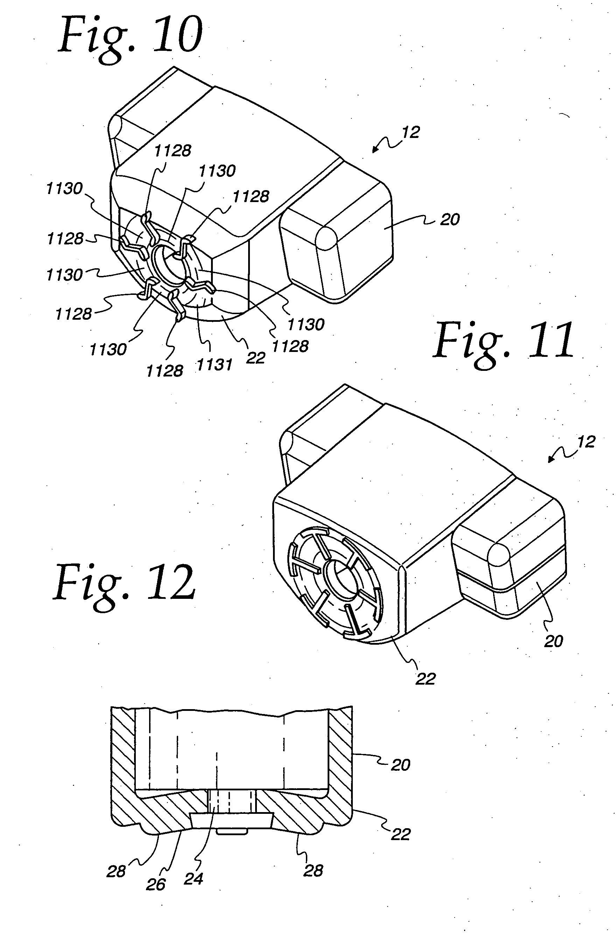

A further aid to blood flow is a concave configuration of the face 22 (FIG. 12). The face 22 has a five to six degree angle from the outer edge to the center aperture 24. Although the face 22 can be flat, the concave configuration helps in pushing the blood toward the center aperture 24. Preferably, the ribs or pressure points 28 are parallel to and follow the concave surface.

While massaging the puncture site the user should observe the puncture site to make sure contact of the face 26 with the skin is maintained and to determine when a sufficient blood sample has been obtained. To allow these observations, the base cap 20 and the top cap 22 can be formed of transparent material. Once a sufficient sample of blood is at the puncture, the lancing device 10 can be lifted from the site and a testing instrument applied to the blood sample.

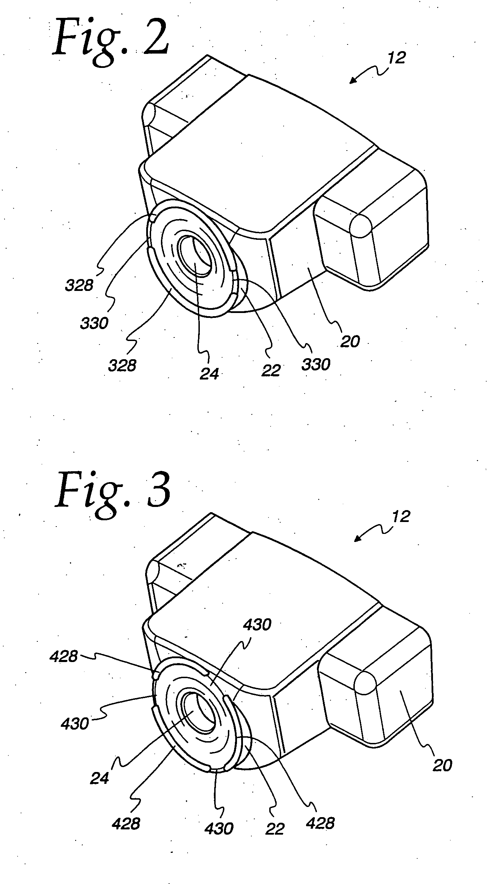

Various patterns of ribs are illustrated in FIGS. 2-11 and these patterns will be briefly described. The endcaps, base caps and top caps in each of FIGS. 2-11 are identical to the endcap 12, base cap 20 and top cap 22 of FIG. 1. For this reason, these same reference numerals appear in FIGS. 2-11 for the end caps, base caps and top caps.

The rib pattern of FIG. 2 consists of two semicircular ribs 328 that are concentric with the central aperture 24. Gaps or channels 330 are defined between adjacent ends of the semicircular ribs 328.

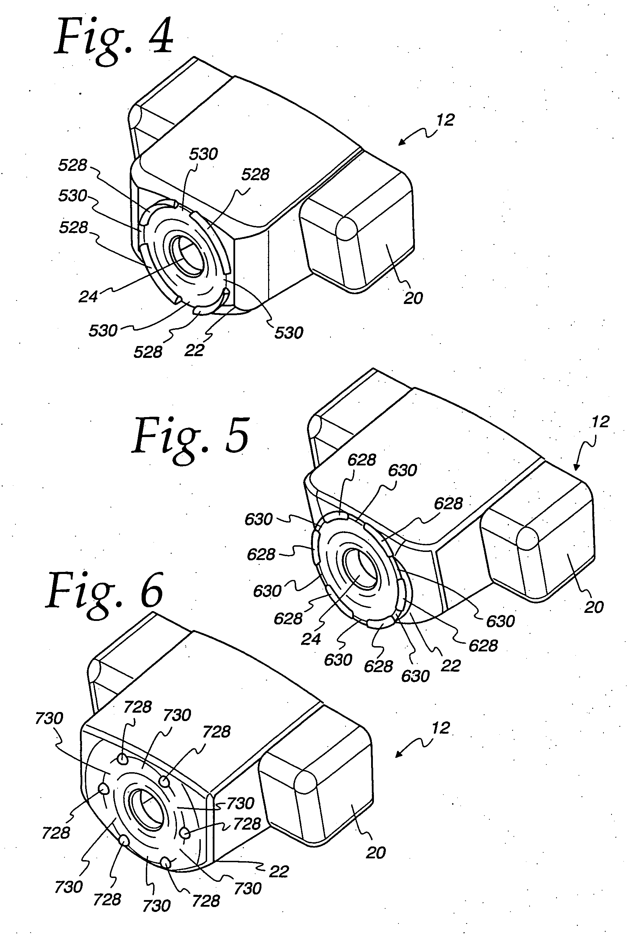

The pattern illustrated in FIG. 3 is three radial ribs 428 with three gaps or channels 430 defined between the ends of adjacent ribs 428. The ribs 428 are concentric with the central aperture 24. The rib pattern in FIG. 4 is similar to the pattern of FIG. 3 but with four radial ribs 528 concentric with the central aperture 24 and defining gaps or channels 530. Similarly, in FIG. 5 there is illustrated a pattern of six radial ribs 628 ad six gaps or channels 630 all concentric with the central aperture 24.

The end cap 12 in FIG. 6 differs from the end caps 12 of FIGS. 1-5 in that there is a pattern of six spherical ribs 728 with gaps or channels 730 defined between the spherical.ribs 728.

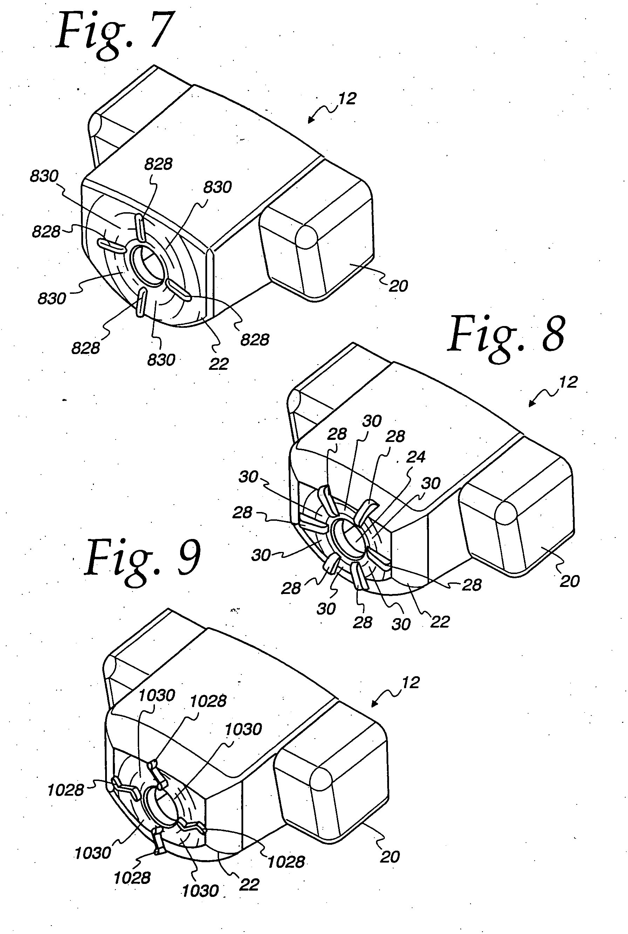

The pattern in FIG. 7 is similar to the pattern in FIG. 1 except the pattern in FIG. 7 includes four radial ribs 828 with four gaps or channels 830.

FIG 8 is an isometric illustration of the end cap 12 of FIG. 1 shown separately and detached from the lancing device 10. The end cap 12 of FIG. 9 is similar to the end cap of FIG. 7 except the four ribs 1028 are S shaped and define gaps or channels 1030 between adjacent ribs 1028. Similarly, the pattern of FIG. 10 includes six S shaped ribs 1128 and six channels 1130.

Another variation of the rib pattern is illustrated in FIG. 11. This pattern includes six T shaped ribs 1228 with six channels 1230.

While the invention is susceptible to various modifications and alternative forms, specific embodiments thereof have been shown by way of example in the drawings and herein described in detail. It should be understood, however, that it is not intended to limit the invention to the particular forms disclosed, but on the contrary, the intention is to cover all modifications, equivalents, and alternatives falling within the spirit and scope of the invention as defined by the appended claims.

## Claims

1. An endcap for a lancing device comprising:
a body,
a contact face on said body
an opening in said contact face, and
a pattern of pressure points on said face, wherein said pressure points comprise a pattern of a plurality of ribs with channels defined between adjacent ribs.

2. The endcap claimed in claim 1 wherein said body is formed of transparent material.

3. The endcap claimed in claim 1 wherein said contact face is concave.

4. The endcap claimed in claim 1 wherein said pressure points are concentric with said opening.

5. The endcap claimed in claim 1 wherein said pressure points extend radially from said opening.

6. An endcap for a lancing device comprising:
a body, said body including a face, an aperture in said face for passage of a lancet, said face being concave, and a rib pattern on said face.

7. The endcap claimed in claim 6 wherein said rib pattern includes a plurality of ribs defining channels between said ribs.

8. The endcap claimed in claim 6 wherein said body is fabricated of transparent material.

9. The endcap claimed in claim 6 wherein said rib pattern includes a plurality of ribs concentric with said aperture.

10. The endcap claimed in claim 6 wherein said rib pattern includes a plurality of ribs extending radially from said aperture.

11. A method of drawing a blood sample for testing, comprising:
providing a lancing device having a lancet and an endcap, said endcap including a skin contacting face with a pattern of pressure points on said face,
placing said face of said endcap onto a selected site,
actuating said lancet to puncture skin at said selected site and,
massaging said site with said skin contacting face on said site to cause blood to flow to said puncture.

12. The method of drawing a blood sample for testing claimed in claim 11 further comprising massaging said site with said skin contacting face prior to actuating said lancet.

13. The method of drawing a blood sample for testing claimed in claim 11 wherein massaging said site comprises alternating turning said endcap clockwise and counterclockwise while maintaining pressure on said selected site.

14. The method of drawing a blood sample for testing claimed in claim 11 wherein said massaging said site comprises rocking said skin contacting face on said site.

15. The method of drawing a blood sample for testing claimed in claim 11 wherein said massaging said site comprises moving said skin contacting face toward and away from said site.

16. The method of drawing a blood sample for testing claimed in claim 11 wherein said massaging said site comprises applying constant pressure on said skin contacting face to move said skin contacting face against said site and apply constant pressure thereon.

17. The method of drawing a blood sample for testing claimed in claim 11 further comprising forming said pattern of pressure points to define channels in said skin contacting face between said pressure points.

18. The method of drawing a blood sample for testing claimed in claim 11 wherein said skin contacting face includes an aperture for passage of said lancet, further comprising beveling said skin contacting face around said aperture thereby providing gradual reduction of pressure toward said aperture as said site is massaged by said skin contacting face.

19. The method of drawing a blood sample for testing claimed in claim 11 wherein said endcap is transparent, further comprising viewing said selected site through said transparent endcap while massaging said site to determine the amount of blood for testing.
